# EUROPEAN PATENT SPECIFICATION

(11) **EP 1 534 688 B1**
(45) Date of publication and mention of the grant of the patent: **29.10.2014**
(21) Application number: 03788181.0
(22) Date of filing: 15.08.2003
(51) Int. Cl.: C08F 246/00, C08G 83/00, C07D 239/47, C07D 239/48

(54) **MONOMERS CAPABLE OF FORMING FOUR HYDROGEN BRIDGES AND SUPRAMOLECULAR POLYMERS FORMED BY COPOLYMERIZATION OF THESE MONOMERS WITH REGULAR MONOMERS**
MONOMERE, DIE DAZU IN DER LAGE SIND, VIER WASSERSTOFFBRÜCKEN AUSZUBILDEN, UND DURCH COPOLYMERISIERUNG DIESER MONOMERE MIT REGULÄREN MONOMEREN GEBILDETE SUPRAMOLEKULARE POLYMERE
MONOMÈRES POUVANT FORMER QUATRE LIAISONS HYDROGÈNE ET POLYMÈRES SUPRAMOLÉCULAIRES FORMÉS PAR COPOLYMÉRISATION DE CES MONOMÈRES AVEC DES MONOMÈRES RÉGULIERS

(30) Priority: 16.08.2002 US 403636 P
(43) Date of publication of application: 01.06.2005
(73) Proprietor: SupraPolix B.V., 5612 AX Eindhoven (NL)
(72) Inventor: JANSSEN, Henricus, Marie, NL-5625 AM Eindhoven (NL); VAN GEMERT, Gaby, Maria, Leonarda, NL-6041 GH Roermond (NL); TEN CATE, Aafke, Tessa, NL-5216 KE Den Bosch (NL); VAN BEEK, Dimphna, Johanna, Maria, NL-5615 CX Eindhoven (NL); SIJBESMA, Rintje, Pieter, NL-5212 AK Den Bosch (NL); MEIJER, Egbert, Willem, NL-5583 GC Waalre (NL); BOSMAN, Anton, Willem, NL-5613 LK Eindhoven (NL)
(74) Representative: Nederlandsch Octrooibureau
(86) International application number: PCT/NL2003/000586
(87) International publication number: WO 2004/016598

(56) References cited:
- EP-A- 0 624 605
- FR-A- 1 350 973
- US-A- 2 461 943
- US-A- 2 694 687
- US-A- 2 744 943
- US-B1- 6 320 018
- YAMAUCHI, K; LIZOTTE, J.R., LONG, T.E.: "Thermoreversible poly(alkyl acrylates) consisting of self-complementary multiple hydrogen bonding" MACROMOLECULES, vol. 36, no. 4, 2003, pages 1083-1088, XP002260387
- RIETH, L.R., EATON, R.F., COATES, G.W.: "Polymerization of ureidopyrimidinone-functionalized olefines by using late-transition metal Ziegler-Natta catalysts: synthesis of thermoplastic elastomeric polyolefins" ANGEW. CHEM. INT. ED. , vol. 40, no. 11, 2001, pages 2153-2156, XP002260388 Weinheim cited in the application
- HERWEH, J.E. & WHITMORE, W.Y.: "Synthesis and Characterization of 2-Ureylene-4,6-diamino-s-triazines" J. CHEM. ENG. DATA, vol. 15, no. 4, 1970, pages 593-595, XP002260389
- PATENT ABSTRACTS OF JAPAN vol. 001, no. 012 (C-004), 22 March 1977 (1977-03-22) -& JP 51 125092 A (TOYAMA CHEM CO LTD), 1 November 1976 (1976-11-01)
- EL-GHAYOURY, A. ET AL.: "Supramolecular hydrogen-bonded oligo(p-phenylene vinylene) polymers" ANGEW. CHEM. INT. ED., vol. 40, no. 19, 2001, page 3660-3663 XP002260390
- EL-GHAYOURY, A. ET AL.: "Quadruple hydrogen bonded oligo(p-phenylene vinylene) dimers" CHEM. COMMUN., 2000, pages 1969-1970, XP002260391

## Description

### FIELD OF THE INVENTION

The invention relates to the synthesis of polymers containing self-complementary quadruple hydrogen groups by copolymerizing monomers containing a quadruple hydrogen bonding group with one or more monomers of choice. The resulting polymers show unique new characteristics due to the presence of additional physical interactions between the polymer chains that are based on multiple hydrogen bonding interactions (supramolecular interactions).

### BACKGROUND OF THE INVENTION

This invention relates to polymers containing units that are capable of forming H-bridges with each other leading to physical interactions between different polymer chains. The physical interactions originate from multiple hydrogen bonding interactions (supramolecular interactions) between self-complementary units containing at least four hydrogen bonds (units capable of forming at least four hydrogen bonds are in this application abbreviated as 4H-units or 4H-monomers and are used in this application as interchangeable terms) in a row. Sijbesma et al. (US 6.320.018; Science, 278, 1601) discloses such self-complementary units which are based on 2-ureido-4-pyrimidones. In Examples X the 4H-unit 6-(3-butenyl)-2-butylureido-4-pyrimidone is disclosed. Polymers obtained by polymerization of the carbon-carbon double bond moiety of this compound are, however, not disclosed.

Telechelic polymers have been modified with 4H-units (Folmer, B.J.B. et al., Adv. Mater. 2000, Vol. 12, 874; Hirschberg et al., Macromolecules 1999, Vol. 32, 2696). However, this has been performed after polymerization in a laborious post-modification process. Another drawback of these polymers containing 4H-units is that they only contain the 4H-unit coupled at the ends of the polymers. Consequently, the number of end groups is therefore limited by the amount of end groups (normally 2), and the functional units are always located on the periphery of the polymer.

Polymers containing hydrogen bonding groups in the main chain synthesized via copolymerization of hydrogen bonding monomers have been obtained with hydrogen bonding units containing three H-bonds in a row (Lange F.M. et al., Macromolecules 1995, Vol 28, 782). However, only an alternating copolymer of styrene and maleimide can be used in this approach, and moreover, the H-bonding interactions between the polymers are much weaker than the H-bonding based on the 4H-units, obviously resulting in poorer material properties.

Polymers with quadruple H-bonding units in the main chain have been obtained by copolymerizing 4H-monomers in the main chain of a polyolefin (Rieth, L.R. et al., Angew. Chem. Int. Ed., 2001, Vol. 40, 2153). However, complex chemistry has to be used to prepare and to polymerize the monomer and, due to the intrinsic sensitivity of the catalyst needed to obtain the polymer, severe limitations hinder the general use of this system and limits it to tailor-made polyolefin systems. For example, Coates et al. discloses the copolymerization of 1-hexene and a 6-hexenyl-2-ureido-4-pyrimidone derivative with a Ziegler-Natta type nickel based catalyst and diethylaluminum chloride as cocatalyst.

EP 624.605 A2, Example 1, discloses the 1:1 adduct of isopropenyl-α,α-dimethylbenzy isocyanate (TMI) and melamine which has the formula:

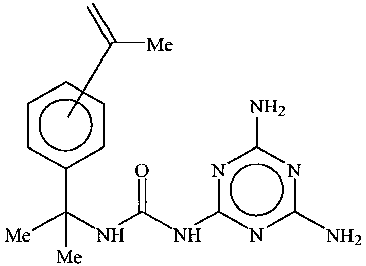

Herweh, J.E. and Whitmore, W.Y, J Chem. Eng. Data, 1970, Vol. 15, 193, discloses a 2-ureylene-4,6-diamino-s-triazine having an allyl group (compound IIb):

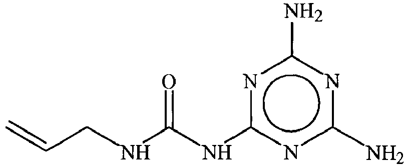

JP S 51125092 A discloses s-triazines containing the group -NHCH₂CH=CH₂, e.g. N-(4-chloro-6-(2-propenylamino)-1,3,5-triazin-2-yl)-N'-ethylurea RN 62734-51-4 and N-(4-methoxy-6-(2-propenylamino)-1,3,5,-triazin-2-yl)-N'-ethylurea RN 62734-61-6.

El-ghayoury, A., et al;. Angew. Chem. Int. Ed., 2001, Vol. 40, 3660, discloses the compounds MOPVUP and BOPVUP wherein a 4H-unit is covalently bonded to an oligo-para-phenylene-vinylene (OPV) derivative. These compounds contain carbon carbon double bonds which are, however, not reactive towards polymerization.

El-ghayoury, A., et al; Chem. Commun. 2000, 1969, discloses the compounds OPV3UP, OPV4P1 and OPVUP2 wherein a 4H-unit is covalently bonded to an oligo-para-phenylene-vinylene (OPV) derivative. These compounds are similar to the compounds MOPVUP and BOPVUP.

US 2.694.687 discloses vinyloxyalkylmelamines according to the formula: 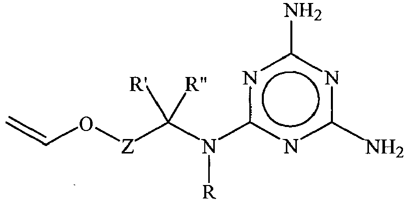 wherein R is hydrogen or an hydrocarbon group containing up to eighteen carbon atoms and wherein R' and R" are hydrogen or an alkyl group.

US 2.744.943 discloses methacrylamidoacylguanamines according to the formula: 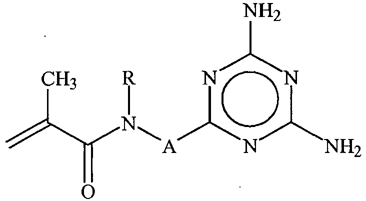 wherein A is an alkylene group containing one to eight carbon atoms and R is hydrogen, alkyl, alkenyl, aralkyl or cycloalkyl.

FR A 1.350.973 discloses compounds according to formula (III): 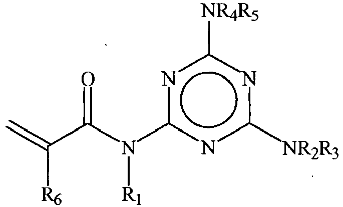 wherein R₁, R₂, R₃, R₄ and R₅ are independently (cyclo)aliphatic groups, araliphatic groups, aromatic groups or heterocyclic groups, R₁ is hydrogen and R₆ is hydrogen or alkyl.

US 2.461.943 discloses guanamines of the formula: 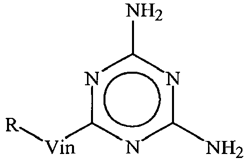 wherein Vin represents a vinyl group and R is an aliphatic radical.

US 3.290.350 discloses α, α, α', α'-tetramethylxylylene diisocyanates and α, α-dimethylisopropenylbenzyl isocyanates and the preparation thereof from isocyanic acid and alkenes.

The present invention discloses a convenient synthesis and convenient copolymerization of monomers containing a 4H-unit with other widely available monomers. The present invention can be used for the preparation of a wide range of polymers with 4H hydrogen bonding units in order to provide these polymers with unique new material properties as a result of the incorporation of the 4H-units. These new material properties result from the reversible nature of H-bonding interactions between the polymer chains that allow reversible changing of the material properties by external stimuli like heat or dilution. Consequently, it becomes possible to prepare materials that combine the mechanical properties of conventional macromolecules with the low melt viscosity of organic compounds.

### SUMMARY OF THE INVENTION

According to the invention, the monomers comprise (a) a monomeric unit having a group that can be polymerized (i.e. a monomeric unit having a polymerizable group) and having at least a functional group, (b) a linking moiety and (c) a structural element capable of forming at least four hydrogen bridges, preferably four hydrogen bridges, wherein the monomer has the general structure (a) - (b) - (c), wherein the structural element (c) has the general formula (3) or (4), or tautomers thereof: 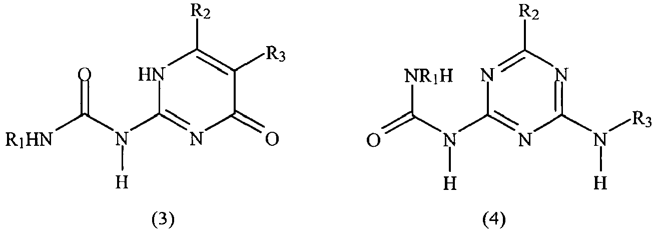 wherein the structural element (c) is bonded to the linking moiety (b) at R₁ with the other R groups representing a random side chain or are hydrogen atoms and wherein the functional group of the monomeric unit having a polymerizable group is selected from the group consisting of hydroxy, carboxylic acid, carboxylic ester, carboxylic acid halide, isocyanate, thioisocyanate, primary amine, secondary amine and halogen.

### DETAILED DESCRIPTION OF THE INVENTION

### Description of the monomer containing the 4H-unit

The monomer containing the 4H-unit comprises a group that can be polymerized, a linker and a 4H-unit. In particular, the group that can be polymerized is linked to a 4H-unit via a linker as is shown below in schematic form.

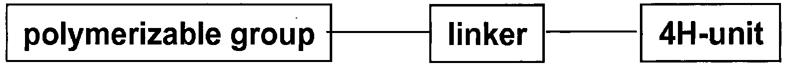

According to the invention, the monomers comprise (a) a monomeric unit having a group that can be polymerized (i.e. a monomeric unit having a polymerizable group), (b) a linking moiety and (c) a structural element capable of forming at least four hydrogen bridges, preferably four hydrogen bridges, wherein the monomer has the general structure:

(a) - (b) - (c)

Preferably, (a) comprises monomeric units having an ethylenically unsaturated group or an ion-polymerizable group. Most preferably, group (a) comprises monomeric units having an ethylenically unsaturated group.

The structural element (c) according to the invention are the compounds shown below having general formulae (3) or (4), and tautomers thereof:

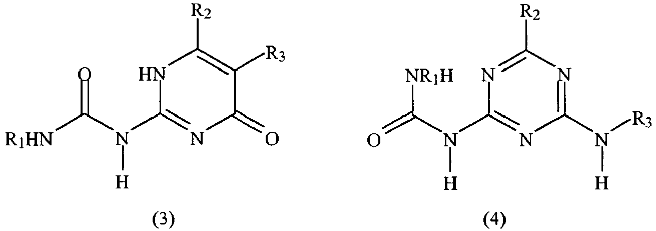

The structural element (c) is bonded to the linking moiety (b) at R₁ (so that R₁ represents a direct bond) whereas R₂ and R₃ are a random side chain or are hydrogen atoms. Most preferably, R₂ is a random side chain and R₃ a hydrogen atom, wherein the random side chain is an alkyl or oligoethylene glycol group in the 6-position, most preferably methyl, or ethylhexyl.

The linking moiety (b) may be all kinds of shorter or longer chains, for example saturated or unsaturated, branched, cyclic or linear alkyl chains, siloxane chains, ester chains, ether chains and any chain of atoms used in traditional polymer chemistry, whether or not substituted with functional groups such as esters, ethers, ureas or urethanes. Preferably, the linking moiety (b) is a C₁-C₂₀ straight chain or branched alkylene, arylene, alkarylene or arylalkylene group, more preferably a C₂-C₁₀ straight chain or branched alkylene, arylene, alkarylene or arylalkylene group, wherein the alkylene, arylene, alkarylene or arylalkylene group may be substituted with other groups or may contain cyclic groups as substituent or in the main chain. Examples of such groups are methylene, ethylene, propylene, tetramethylene, pentamethylene, hexamethylene heptamethylene, octamethylene, nonamethylene, 1,6-bis(ethylene)cyclohexane, and 1,6-bismethylene benzene. The alkylene, arylene, alkarylene or arylalkylene groups may be interrupted by heteroatoms, in particular heteroatoms selected from the group of oxygen, nitrogen, and sulphur. The linking moiety (b) that links the monomeric unit having a polymerizable group (a) to structural element (c) is derived from a compound that must have at least two functional groups, wherein the functional groups are selected from the group consisting of hydroxy, carboxylic acid, carboxylic ester, carboxylic acid halide, isocyanate, thioisocyanate, primary amine, secondary amine, and halogen. These functional groups are preferably present as end groups. According to the invention, such preferred compounds from which the linking moieties (b) are derived are preferably those having isocyanate or thioisocyanate end groups, more preferably isocyanate end groups. Most preferably, these compounds are diisocyanates or dithioisocyanates, in particular diisocyanates. Examples of suitable diisocyanates that can be used in this invention are:
1,4-diisocyanato-4-methyl-pentane,
1,6-diisocyanato-2,2,4-trimethylhexane,
1,6-diisocyanato-2,4,4-trimethylhexane,
1,5-diisocyanato-5-methylhexane,
3((4)-isocyanatomethyl-1-methylcyclohexyl isocyanate,
1,6-diisocyanato-6-methyl-heptane,
1,5-diisocyanato-2,2,5-trimethylhexane,
1,7-diisocyanato-3,7-dimethyloctane,
1-isocyanato-1-methyl-4-(4-isocyanatobut-2-yl)-cyclohexane,
1-isocyanato-1,2,2-trimethyl-3-(2-isocyanato-ethyl)-cyclopentane,
1-isocyanato-1,4-dimethyl-4-isocyanatomethyl-cyclohexane,
1-isocyanato-1,3-dimethyl-3-isocyanatomethyl-cyclohexane,
1-isocyanatol-n-butyl-3-(4-isocyanatobut-1-yl)-cyclopentane.
1-isocyanato-1,2-dimethyl-3-ethyl-3-isocyanatomethyl-cyclopentane,
3((4)-isocyanatomethyl-1-methylcyclohexyl isocyanate (IMCI),
toluene diisocyanate (TDI),
methylene diphenyl diisocyanate (MDI),
methylene dicyclohexane 4,4-diisocyanate,
isophorone diisocyanate (IPDI), hexane diisocyanate (HDI).

Examples of suitable thioisocyanates are the dithioisocyanate derivatives of the compounds exemplified above for suitable dithiocyanates.

Preferably, the diisocyanate is IPDI, HDI, MDI, TDI or methylene dicyclohexane 4,4-diisocyanate and their thioisocyanate counterparts. According to the invention, however, the diisocyanates are more preferably used than dithioisocyanates.

The monomeric unit having a polymerizable group (a) comprises preferably monomeric units having an ethylenically unsaturated group or an ion-polymerizable group and most preferably the monomeric unit having a polymerizable group comprises a monomeric unit having an ethylenically unsaturated group, i.e. a group derived from monomers having a carbon carbon double bond. According to a preferred embodiment of the invention, the monomeric unit having a polymerizable group has at least one functional group selected from the group consisting of hydroxy, carboxylic acid, carboxylic ester, carboxylic acid halide, isocyanate, thioisocyanate, primary amine, secondary amine and halogen. According to a more preferred embodiment of the invention, the monomeric unit having a polymerizable group is derived from acrylates, methacrylates, acrylamides, methacrylamides, styrenes, vinyl-pyridines, other vinyl monomers, lactones, other cyclic esters, lactams, cyclic ethers and cyclic siloxanes. According to the most preferred embodiment of the invention, the monomeric unit having a polymerizable group is derived from acrylates, methacrylates and vinyl esters, most preferably vinyl acetates. Examples of compounds from the monomeric units having a polymerizable group that are in particular useful in carrying out the invention are: 2-hydroxyethyl acrylate, 2-hydroxy-propyl acrylate, 2,3-dihydroxypropyl acrylate, poly(ethylene glycol) acrylate, acrylamide, N-hydroxymethyl acrylamide, 2-hydroxyethyl methacrylate, 2-hydroxy-propyl methacrylate, 2,3-dihydroxypropyl methacrylate, poly(ethylene glycol) methacrylate, N,N-dimethylaminoethylmethacrylate, N-hydroxymethyl methacrylamide, vinylacetate, 4-vinyl phenol, 4-vinyl aniline, 4-hydroxymethyl-styrene, 4-aminomethyl-styrene, 4-(2-hydroxyethyl)-s-caprolactone, 4-(4-hydroxyphenyl)-s-caprolactone, and 2,3-epoxy-1-propanol.

According to the invention, the monomers are preferably prepared by the following methods.

According to a first method, the monomeric unit having a polymerizable group is reacted in a first step with the compound that must have at least two functional groups. In a subsequent step, the product obtained in the first step is reacted with the nitrogen containing compound. Suitable and preferred structures of the monomeric unit having a polymerizable group, the compound that must have at least two functional groups and the nitrogen containing compound are described above.

According to a second method, the nitrogen containing compound is reacted in a first step with the compound that must have at least two functional groups. In a subsequent step, the product obtained in the first step is reacted with the monomeric unit having a polymerizable group.

According to a third method, the nitrogen containing compound is reacted directly with the monomeric unit having a polymerizable group wherein the monomeric unit is able to form a urea linkage between both reactants.

According to these methods, the monomeric unit having a polymerizable group is most preferably selected from the group of monomeric units having an ethylenically unsaturated group, in particular monomers having a carbon carbon double bond, wherein the monomeric unit having a polymerizable group has preferably at least one functional group, wherein the functional group is selected from the group of hydroxy, carboxylic acid, carboxylic ester, carboxylic acid halide, isocyanate, thioisocyanate, primary amine, secondary amine or halogen groups. More preferably, the monomeric unit having a polymerizable group is selected from the group of acrylates, methacrylates, acrylamides, methacrylamides, styrenes, vinyl-pyridines, other vinyl monomers, lactones, other cyclic esters, lactams, cyclic ethers and cyclic siloxanes having a functional group selected from hydroxy, carboxylic acid, carboxylic ester, isocyanate, thioisocyanate, primary amine, secondary amine or halogen groups. Even more preferably, the monomeric unit having a polymerizable group is selected from the group of acrylates, methacrylates and vinyl esters, in particular vinyl acetates, said acrylates, methacrylates and vinyl esters having preferably a functional group selected from hydroxy, carboxylic acid, carboxylic ester, carboxylic acid halide, isocyanate, thioisocyanate, primary amine, secondary amine or halogen groups.

Preferred embodiments of the methods for the preparation of the monomers are shown below in Schemes 1-3. 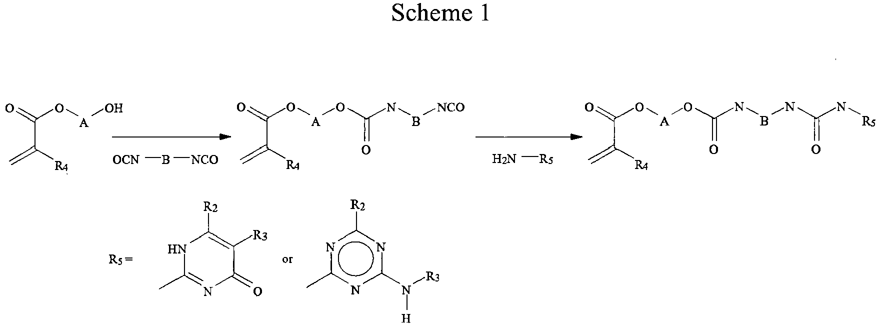 wherein R₂ and R₃ are as defined above, R₄ is hydrogen or methyl, A is a chain, preferably an oligomethylene chain or an oligoethylene glycol chain (as will be appreciated by the person skilled in the art, A may be absent so that the monomeric unit having a polymerizable group has a carboxylic group as functional group) and B is the chain of the linking moiety (b) described above.

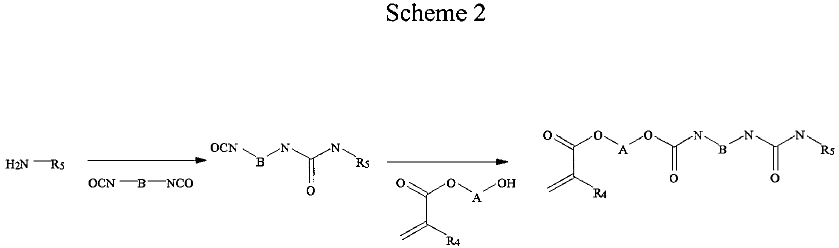

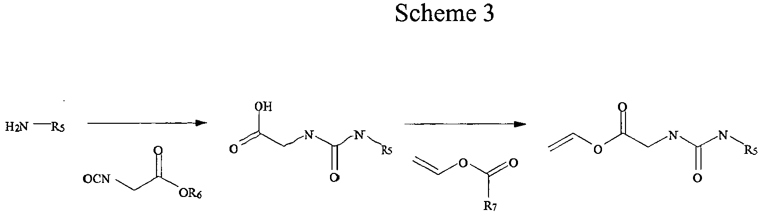

In Scheme 3 R₆ and R₇ represent each independently a C₁-C₆ alkyl group, wherein R₇ is preferably methyl.

### Description of the co-polymerization and of the polymer

The polymers presented in this invention are obtained by co-polymerizing the monomer containing the 4H-unit with one or more, optionally different comonomers that can be from the same family or from a different family of monomers. These comonomers are preferably selected from the group of: acrylic acid; C₁-C₃₀ branched or linear alkyl esters of acrylic acid; methacrylic acid; C₁-C₃₀ branched or linear alkyl esters of methacrylic acid; acrylamides or methacrylamides wherein the amide group may be substituted with one or two C₁-C₃₀ branched or linear alkyl groups; vinyl esters, preferably vinyl acetates; other compounds having a vinyl group wherein said compounds are preferably selected from pyrrolidones, imidazoles, pyridines, caprolactams, piperidones, benzene: and derivatives thereof; C₄-C₂₀ alkadienes; lactones; lactams; and saturated or unsaturated heterocyclic compounds containing one to five oxygen atoms. Examples of suitable comonomers are acrylic acid, methyl acrylate, butyl acrylate, 2-ethylhexyl acrylate, 2-hydroxyethyl acrylate, N,N-dimethylacrylamide, N-isopropylacrylamide, methacrylic acid, methyl methacrylate, ethyl methacrylate, butyl methacrylate, isobutyl methacrylate, 2-ethylhexyl methacrylate, lauryl methacrylate, 2-hydroxy-ethyl methacrylate,, vinylacetate, N-vinylpyrrolidinone, 2-vinylpyridine-1-oxide, N-vinyl imidazole, N-vinyl pyridine, N-vinylcaprolactam, N-vinyl-2-piperidone, acrylonitrile, styrene, butadiene, isoprene, caprolacton, butyrolacton, caprolactam, ethyleenoxide, propyleneoxide, tetrahydrofuran, 3,6-dimethyl-1,4-dioxane-2,5-dione, 1,4-dioxane-2,5-dione.

The copolymerizations may be of any type (for example, bulk, dispersion, solution, emulsion, suspension or inverse phase emulsion) and of any mechanism (for example, radical polymerization, condensation polymerization, transition metal catalyzed polymerization, enzymatic polymerization, or ring opening polymerization).

The copolymer backbone acquired may be of any type (linear, branched, star, hyperbranched, dendritic, comb-like or the like).

The product copolymer may be of any structure. For example random, regular, tapered or block copolymer structures are allowed.

According to the invention, the molecular weights of the polymers are preferably not too high. A preferred number average molecular weight range is 500 - 20000.

The copolymers according to the invention are in particular suitable for applications related to personal care (hair preparations, skin cosmetics and laundry aids), surface coatings (leather, textile, optical fibers, paper and paint formulations), imaging technologies (printing, stereolithography, photography and lithography), biomedical applications (materials for controlled release of drugs and materials for tissue-engineering, tablet formulation), adhesive and sealing compositions, and thickening agent and binders.

### EXAMPLES

The following non-limiting examples further illustrate the preferred embodiments of the invention. When not specifically mentioned, chemicals are obtained from Aldrich.

### Synthesis of building blocks

### Example 1: synthesis of isocyanate I

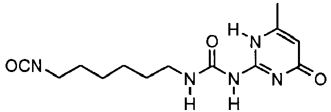

1,6-Hexyldiisocyanate (650 g) and methyl isocytosine (or 2-amino-4-hydroxy-6-methyl-pyrimidine, 65.1 g) were suspended in a 2-liter flask. The mixture was stirred overnight at 100°C under an argon atmosphere. After cooling to room temperature, a liter of pentane was added to the suspension, while stirring was continued. The product was filtered, washed with pentane and dried in vacuum. A white powder was obtained. ¹H NMR (400 MHz, CDCl₃): δ 13.1 (1H), 11.8 (1H), 10.1 (1H), 5.8 (1H), 3.3 (4H), 2.1 (3H), 1.6 (4H), 1.4 (4H). FT-IR (neat): v 2935, 2281, 1698, 1668, 1582, 1524, 1256.

### Example 2: synthesis of isocyanate II

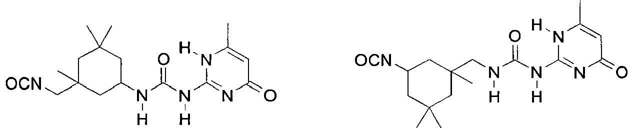

Methyl isocytosine (5.2 g) was added to isophoronediisocyanate (IPDI, 50 mL) and subsequently stirred at 90°C under an argon atmosphere for 3 days. The resulting clear solution was precipitated in heptane. The white gum was collected, heated in 150 mL heptane, cooled on ice, and filtered. The same procedure was repeated once more with the white residue, resulting in a white powder. ¹H NMR (400 MHz, CDCl₃): δ 13.1 (1H), 12.0 (1H), 10.1 (1H), 5.9 (1H), 4.1-3.1 (3H), 2.1 (3H), 2.0-0.9 (15H). FT-IR (neat): v (cm⁻¹) 2954, 2255, 1696, 1662, 1582, 1524, 1247.

The product exists in four different isomers: the two regio-isomers depicted above are both present in cis and trans configuration. For reasons of clarity, only one isomer is depicted in the following schemes, although all four isomers are present.

### Example 3: synthesis of isocyanate III

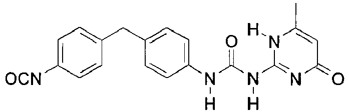

Methyl isocytosine (2.0 g) was added to 4,4'-methylenebis(phenylisocyanate) (MDI, 8.1 g) dissolved in 35 mL THF and subsequently stirred at an oil bath temperature of 75°C under an argon atmosphere. After 4 hours, 40 mL chloroform was added and the white precipitate was separated by filtration and washed with chloroform. ¹H NMR (400 MHz, DMSO-d6): δ 10.0 (1H), 8.6 (1H), 7.4 (5H), 7.2 (4H), 5.8 (1H), 3.8 (2H), 2.1 (3H). FT-IR (neat): v (cm⁻¹) 3329, 2954, 2257, 1699, 1658, 1578, 1508, 1244.

### Example 4: synthesis of isocyanate IV

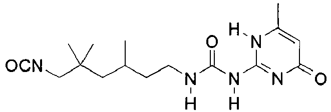

A suspension of methyl isocytosine (2 g) and trimethyl-1,6-hexyldiisocyanate (a mixture of 2,2,4-trimethyl and 2,4,4-trimethyl isomers; 24 g) was stirred and heated for 21 hours at an oil bath temperature of 100°C. The mixture was kept under an argon atmosphere. The reaction mixture was cooled down to room temperature and pentane was added to induce precipitation. The suspension was filtrated over a glass filter and the gummy residue was stirred and heated again in pentane. Cooling, filtration and drying gave a white powder. ¹H NMR (300 MHz, CDCl₃): δ = 13.1 (1H), 11.7 (1H), 10.1 (1H), 5.8 (1H), 3.4-3.0 (4H), 2.2 (3H), 1.8 (1H), 1.6 (2H), 1.3 (1H), 1.1 (1H), 1.05-0.95 (9H). FT-IR (neat): υ (cm⁻¹) 2933, 2260, 1693, 1647, 1580, 1518, 1248.

The product exists in eight isomers, as both trimethyl hexyldiisocyanates are chiral and can react at both isocyanate positions. For reasons of clarity, only one isomer is depicted (without assignment of stereocenter), although all isomers are present.

### Example 5: synthesis of 6-(1-ethylpentyl)-isocytosine

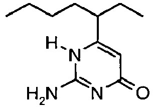

Potassium ethyl malonate (150 g) and acetonitrile (1.4 L) were stirred in a flask and brought to a temperature of 10-15°C. Triethylamine (132 mL) was added drop wise, while keeping the mixture under an argon atmosphere. Dried MgCl₂ (101.6 g) was added and the suspension was stirred for 2 hours at room temperature. Thereafter, the suspension was cooled to 0°C and 2-ethylhexanoyl chloride (74 mL) was added drop wise, and the mixture was allowed to warm up to room temperature and was stirred overnight. The acetonitrile was removed by evaporation, 400 mL toluene was added and evaporated, 700 mL of toluene was added and the mixture was cooled to 10°C. An aqueous HCl solution was added slowly, and the organic layer was separated, washed with an HCl solution and then with a bicarbonate solution. The organic layer was dried with Na₂SO₄ and concentrated to give the β-ketoester as a liquid. The β-ketoester (50 g) and guanidine carbonate (49.8 g) were boiled in ethanol (300 mL) for two days using a Soxhlett set-up with molsieves in the thimble. The suspension was filtered, ethanol was evaporated and the product was dissolved in chloroform. After washing with a bicarbonate solution, the organic layer was dried with MgSO₄, concentrated and dropped into an excess of pentane to yield a white powder. ¹H NMR (400 MHz, CDCl₃): δ 11.6-10.6 (1H), 7.6-6.6 (2H), 5.6 (1H), 2.2 (1H), 1.5 (4H), 1.2 (4H), 0.8 (6H). FT-IR (neat): v 3322, 3152, 2929, 2860, 1635, 1463, 1378, 1582, 1524.

### Synthesis of 4H hydrogen bonding unit containing monomers

### Example 6: monomer 1, a 4H hydrogen bonding unit containing methacrylate monomer

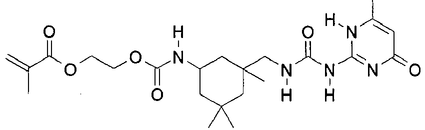

Isocyanate II (28 g) was dissolved in chloroform (0.5 L), and thereafter hydroxy ethyl methacrylate (HEMA, 9.6 mL) and 8 drops of dibutyl tin dilaurate (DBTDL) were added. The mixture was stirred at an oil bath temperature of 90°C for 4 hours, and was then cooled and filtered. The filtrate was concentrated and dropped into an excess of diethyl ether. The precipitate was collected by filtration, and was washed with diethyl ether. Drying in vacuo gave a solid product. ¹H NMR (400 MHz, CDCl₃): δ 13.1 (1H), 11.8 (1H), 10.1 (1H), 6.1 (1H), 5.8 (1H), 5.6 (1H), 5.0 (1H), 4.3 (4H), 4.1-3.6 (1H), 3.1-2.9 (2H), 2.1 (3H), 2.0 (3H), 1.8-1.5 (2H), 1.4-0.8 (13H). FT-IR (neat): v 3212, 2954, 1697, 1660, 1572, 1520, 1242, 1165.

### Example 7: monomer 2, a 4H hydrogen bonding unit containing acrylate monomer

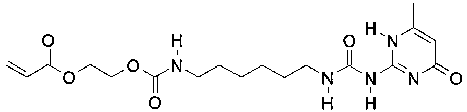

Isocyanate I (46 g) was suspended in chloroform (1 L), and thereafter hydroxy ethyl acrylate (HEA, 36 mL) and 10 drops of dibutyl tin dilaurate (DBTDL) were added. The mixture was stirred at an oil bath temperature of 90°C for 4 hours, and was then cooled and filtered. The filtrate was concentrated and an excess of diethyl ether was added. The white precipitate was collected by filtration, and was washed with diethyl ether. Drying in vacuo gave a white solid product. ¹H NMR (400 MHz, CDCl₃): δ 13.1 (1H), 11.8 (1H), 10.1 (1H), 6.5 (1H), 6.2 (1H), 5.9 (2H), 5.1 (1H), 4.4 (4H), 3.3 (2H), 3.2 (2H), 2.1 (3H), 1.7-1.3 (8H). FT-IR (neat): v 3307, 2928, 1725, 1702, 1682, 1664, 1584, 1548, 1258, 1192.

### Example 8: Monomer 3, a 4H hydrogen bonding unit containing methacrylate monomer

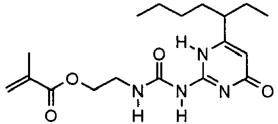

2-Isocyanatoethyl methacrylate (7.0 mL) was added to a solution of 6-(1-ethylpentyl)-isocytosine (13.4 g) in dry pyridine (150 mL). The reaction mixture was stirred under an argon atmosphere at 80°C for 4 hrs. The product was worked-up by evaporation of the solvent, and subsequent filtration over silica using chloroform/methanol (4%). Silica column chromatography using ethyl acetate/hexane yielded a light yellowish waxy solid. ¹H NMR (400 MHz, CDCl₃): δ 13.1 (1H), 12.0 (1H), 10.5 (1H), 6.2 (1H), 5.8 (1H), 5.6 (1H), 4.3 (2H), 3.6 (2H), 2.3 (1H), 1.9 (3H), 1.8-1.5 (4H), 1.4-1.2 (4H), 0.9 (6H). FT-IR (neat): v 2959, 2930, 1720, 1697, 1645, 1582, 1555, 1525, 1462, 1254, 1160.

### Example 9: monomer 4, a 4H hydrogen bonding unit containing methacrylate monomer

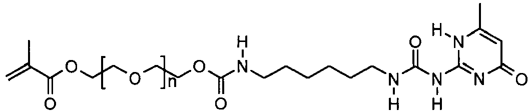

PEG-MA alcohol (with an average molecular weight Mn of 360; 2.2 g), isocyanate I (1.8 g) and a few drops of DBTDL were boiled overnight in chloroform. Hexane was added to cause precipitation. The product was isolated by filtration, washing with hexane and with diethyl ether. ¹H NMR (400 MHz, DMSO-d₆): δ 7.5 (1H), 7.2 (1H), 6.0 (1H), 5.7 (1H), 5.6 (1H), 4.2 (2H), 4.0 (2H), 3.7 (2H), 3.6-3.4 (15H-20H), 3.1 (2H), 2.9 (2H), 2.1 (3H), 1.9 (3H), 1.4 (4H), 1.2 (4H).

### Example 10: Monomer 5, a 4H hydrogen bonding unit containing acrylamide monomer

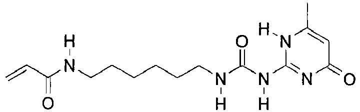

Isocyanate I (1 g) was dissolved in 5 mL of acrylic acid and heated to 60 °C. The mixture was stirred under an argon atmosphere. Copper(II)acetate (8 mg) was added and heating at 60°C was maintained for 2 hours. The product was obtained by precipitation of the reaction mixture into diethyl ether. Then the solid was filtered, dissolved in chloroform and washed with a bicarbonate solution. Drying with Na₂SO₄ and column chromatography using chloroform with 10% methanol gave a white powder. ¹H NMR (400 MHz, CDCl₃/CD₃OD): δ 7.4 (1H), 6.3-6.1 (2H), 5.8 (1H), 5.6 (1H), 3.3-3.2 (4H), 2.1 (3H), 1.7-1.2 (8H). FT-IR (neat): v 3278, 2935, 1699, 1665, 1652, 1582, 1525.

### Example 11: monomer 6, a 4H hydrogen bonding unit containing vinyl-ester monomer

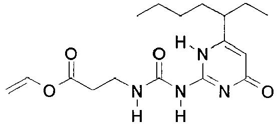

6-(1-Ethylpentyl)-isocytosine (3.5 g) was dissolved in 50 mL THF followed by the addition of 2.5 mL ethyl 3-isocyanato ethylacetate. Stirring the reaction mixture under reflux for 16h resulted in the formation of a white powder that was filtered and washed with chloroform (4.8 gram). This compound was subsequently dissolved in a mixture of ethanol (50 mL) and 1 N aqueous sodium hydroxide (40 mL) and stirred for 2 h at room temperature. Addition of a saturated solution of KHSO₄ in water to the ice-cooled reaction mixture until the pH=3, resulted in the formation of a white precipitate that was subsequently filtrated and washed with water. After drying in vacuo 4.4 gram of the carboxylic acid functional ureidopyrimidone was isolated.

A mixture of the carboxylic acid functional ureidopyrimidone (2.0 gram), mercuric acetate (30 mg), vinylacetate (4.0 gram), and concentrated sulfuric acid (15 uL) was stirred for 2 days at 60 °C. The reaction mixture was subsequently precipitated in diethyl ether. After filtration and drying in vacuo, 4H vinylacetate was obtained as a white powder (2.9 gram).

### Example 12: monomer 7, a 4H hydrogen bonding unit containing styrene monomer

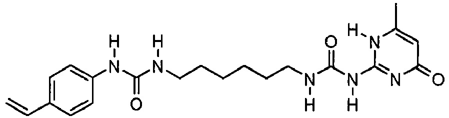

Isocyanate I (5.6 g) was added to 4-aminostyrene (obtained from Aldrich 1.9 g) dissolved in 50 mL of chloroform and subsequently heated to 70°C. The mixture was stirred under an argon atmosphere for 12 h. The product was obtained by separation of the yellowish precipitate from the reaction mixture by filtration, followed by washing with chloroform. ¹H NMR (400 MHz, CDCl₃/DMSO-d6): δ 7.8 (1H), 7.2 (4H), 6.6 (1H), 5.8 (1H), 5.5 (1H), 5.1 (1H), 3.4-3.2 (4H), 2.1 (3H), 1.7-1.2 (8H). FT-IR (neat): v 3278, 2935, 1699, 1665, 1652, 1582, 1525.

### Example 13: monomer 8, a 4H hydrogen bonding unit containing ε-lacton monomer

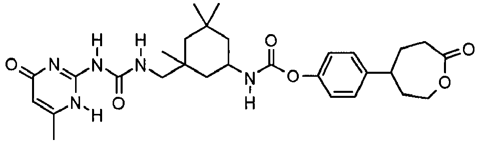

A solution of 4-(4-hydroxyphenyl)-cyclohexanone (obtained from Fluka, 8.2 g) dissolved in 100 mL of 2-butanone was added drop wise to a stirred solution of 3-chloroperoxybenzoic acid (9.3 g) in 40 mL 2-butanone. After 5h the reaction mixture was concentrated in vacuo to 30% of its original volume followed by the addition of 50 mL diethyl ether. The resulting white precipitate was collected by filtration and dried in vacuo. The resulting white powder was redissolved in 150 mL chloroform to which isocyanate II was added (11.8 g) and 4 drops dibutyltinlaureate. The mixture was stirred at reflux under an argon atmosphere for 16h. The product was obtained by precipitation in diethyl ether. ¹H NMR (400 MHz, CDCl₃): δ 13.1 (1H), 12.0 (1H), 10.1 (1H), 7.3-7.0 (4H), 5.9 (1H), 5.4-5.0 (1H), 4.4 (2H), 4.1-3.8 (2H), 3.2-2.6 (4H) 2.2-0.9 (22H). IR (neat): v 3211, 2955, 1724, 1699, 1660, 1575, 1505, 1247, 1211.

### Example 14: monomer 9, a 4H hydrogen bonding unit containing acrylate monomer

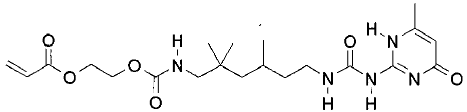

Isocyanate IV (11 g) was dissolved in chloroform (150 mL), and thereafter hydroxy ethyl acrylate (HEA, 4.1 mL) and 4 drops of dibutyl tin dilaurate (DBTDL) were added. The mixture was stirred at an oil bath temperature of 60°C for 9 hours, and was then cooled and precipitated in pentane, followed by a washing step with diethyl ether. The white powder was collected by filtration, and was washed with diethyl ether. Drying in vacuo gave a white solid product. ¹H NMR (400 MHz, CDCl₃): δ 13.1 (1H), 11. 8 (1H), 10.1 (1H), 6.5 (1H), 6.2 (1H), 5.9 (2H), 5.4 and 5.1 (1H), 4.4 (4H), 3.3-2.9 (4H), 2.1 (3H), 1.8-0.9 (14H).

### Example 15: Monomer 10, a 4H hydrogen bonding unit containing methacrylate monomer

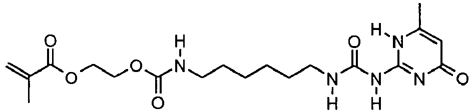

The isocyanate (79 g) was suspended in chloroform (1.5 L), and thereafter hydroxy ethyl methacrylate (HEMA, 64 mL) and 15 drops of dibutyl tin dilaurate (DBTDL) were added. The mixture was stirred at an oil bath temperature of 90°C for 4 hours, and was then cooled and filtered. The filtrate was concentrated and dropped into an excess of diethyl ether. The white precipitate was collected by filtration, and was washed with diethyl ether. Drying in vacuo gave a white solid product (90 g). ¹H NMR (400 MHz, CDCl₃): δ 13.1 (1H), 11.8 (1H), 10.1 (1H), 6.1 (1H), 5.8 (1H), 5.6 (1H), 5.0 (1H), 4.3 (4H), 3.3-3.2 (4H), 2.1 (3H), 1.9 (3H), 1.7-1.2 (8H). FT-IR (neat): v 3301, 2932, 1720, 1699, 1685, 1665, 1582, 1525, 1258.

### Synthesis of co-polymers

Examples of co-polymers containing 4H hydrogen bonding units were obtained via several different polymerization techniques that are known in the art. Results can be found in Table 1, general procedures are given below:

### Atom transfer radical polymerization (ATRP) procedure

### Reparation of poly-HEMA polymers (polymers A-E).

A 25 mL round bottom flask containing CuBr (ca. 1 equiv. to initiator; typically 0.06 to 0.31 g), 2,2'-bipyridine (ca. 2 equiv. to initiator) and the appropriate amount of 4H hydrogen bonding unit containing monomer was degassed (de-oxygenated) by vacuum followed by argon backfill, and repeating this cycle twice. HEMA, DMSO (in equal volumes, typically both 2 to 5 mL) and optionally PEG-MA (average Mₙ = 360 Dalton; for polymers B and C) were degassed (de-oxygenated) by bubbling through argon for at least 45 minutes, and were then added to the 25 mL flask by use of a syringe. The reaction mixture was stirred until all components had dissolved (sometimes after short warming) to produce a homogeneous dark brown solution. The reaction flask was placed in a water bath that was maintained at room temperature, and finally, the ATRP-initiator (benzyl-2-bromo-2-methyl-propionate) was added using a syringe. The molar ratio of ATRP initiator to HEMA was about 1:40 (for polymers A, C, D and E) and about 1:20 for polymer B. Polymerization occurred immediately, leading to an increase in viscosity of the reaction mixture. After ca. half an hour, the polymer was isolated by precipitation into an EDTA (25 g/L) solution in water (polymers B-E), or in chloroform (polymer A). The polymer was washed and dried.

### Preparation of PMMA polymers (polymers F-G).

A flask containing NiBr₂(PPh₃)₂ (ca. 0.35 g) and, optionally, the 4H hydrogen bonding unit containing monomer **3** was degassed (de-oxygenated) by vacuum followed by argon backfill, and repeating this cycle twice. MMA and toluene (in equal volumes; both 5 mL) were degassed (de-oxygenated) by bubbling through argon for at least 45 minutes, and were then added to the flask by use of a syringe. Finally, the ATRP-initiator (benzyl-2-bromo-2-methyl-propionate, ca. 185 mg) was added using a syringe The flask was placed in an oil bath of 70°C and the polymerization was allowed to run for about 20 hours. The solution was filtered over alumina and flushed with chloroform/methanol, the filtrate was concentrated and dropped into hexane. The precipitate was dried.

### Free radical polymerization (FR) procedures (polymers H-L)

A solution containing HEMA (5 mL), the appropriate 4H hydrogen bonding unit containing monomer, AIBN (12 mg) and transfer agent mercapto ethanol (75 microliter; 60 microliter for polymer I) in DMF (15 mL), was degassed by purging with argon for 1 hr prior to polymerization. Polymerization was conducted at 80°C for about 3 to 4 hours, after which the mixture was cooled down to room temperature and the polymer was recovered by precipitation into a non-solvent (THF/hexane 3/1 was used for polymers H and I; water was used for polymers J-L), filtration and drying. For polymer H, different conditions were used: 4.7 mL HEMA, 30 mL DMF, monomer **3**, 20 microliter mercapto ethanol and 22 mg AIBN were stirred at 60°C for two days; the AIBN was added in two equal portions at the beginning and after 1 day of polymerization.

### Ring-opening polymerization (ROP) procedure (polymer M)

Freshly distilled and dried ε-caprolacton, benzylalcohol (initiator) and pyridine (2 equiv. to initiator) were added to a Schlenk bottle in a nitrogen atmosphere that contained tin(II)trifluoromethanesulfonate (catalyst 0.5 equiv. to initiator) and the desired amount of monomer **8**. The Schlenk bottle was placed in an oil bath and heated to 65°C for 16 hours. The viscous reaction mixture was diluted with an equal volume of THF and subsequently precipitated in methanol, filtered and dried in vacuo.

### Emulsion polymerization (EP) procedure (polymers N and O)

Butyl acrylate (5 mL), water (11 mL), optionally monomer **9** (0.67 g), sodium dodecyl sulphate (0.1 g), charge transfer agent dodecyl mercaptane (0.3 mL), initiator sodium persulphate (25 mg for polymer N and 50 mg for polymer O) and sodium bicarbonate (30 mg for polymer N and 25 mg for polymer O) were mixed at 20°C and purged with argon for 45 minutes. Subsequently, the reaction temperature was raised to 50°C and the mixture was extensively stirred for 4 hours. The polymer product was isolated after coagulation of the emulsion by addition of methanol.

**Table 1**

| Polymer | Monomer A | Monomer B | 4H-bridge monomer | Monomer molar Ratio in Feed A : (B) : 4H-mon | method | Mₙ (kDa) from NMR | Mₙ / D (kDa/-) from SEC | # of 4H-bridge monomers per chain |
|---|---|---|---|---|---|---|---|---|
| A | HEMA | - | # 10 | 19.2 : 1 | ATRP | 10 | - | 1.7 |
| B | HEMA | PEG-MA | # 3 | 85 : 6.5 : 8.5 | ATRP | 5.2 | 18/1.8 | 1.8 |
| C | HEMA | PEG-MA | # 3 | 92 : 3.8 : 4.2 | ATRP | 10 | 27 / 1.6 | 2.5 |
| D | HEMA | - | # 3 | 10 : 1 | ATRP | 10 | - | 3.8 |
| E | HEMA | - | # 4 | 21.2 : 1 | ATRP | 6.5 | - | 1.7 |
| F | MMA | - | - | - | ATRP | - | 7.6/1.1 | 0 |
| G | MMA | - | # 3 | 12.9 : 1 | ATRP | - | 9.4 / 1.4 | 3.7 |
| H | HEMA | - | # 3 | 19.4 : 1 | FR | - | - | - |
| I | HEMA | - | # 3 | 37.5 : 1 | FR | - | 24 / 1.4 | - |
| J | HEMA | - | # 10 | 9.6 : 1 | FR | - | - | - |
| K | HEMA | - | # 2 | 9.2 : 1 | FR | - | - | - |
| L | HEMA | - | # 5 | 25.4 : 1 | FR | - | - | - |
| M | ε-CL | - | # 8 | 17 : 1 | ROP | 5.1 | - | 2.8 |
| N | BA | - | # 9 | 23.4 : 1 | EP | - | 15.9* | - |
| O | BA | - | - | - | EP | - | 34.8* | 0 |

| | | | | | | | | |
|---|---|---|---|---|---|---|---|---|
| - = not determined; * Mₜₒₚ (kD) | | | | | | | | |

### Characterization

Molecular weights (Mₙ) of polymers A-E, and M were determined with ¹H NMR by considering the integral of the benzylic end group versus the integral of the repeating unit. The molecular weights (Mₙ) and dispersities (D) of polymers B, C and I were determined with SEC (0.01 M LiBr in DMF) against polystyrene standards (a comparison with the NMR data for polymers B and C shows that the Mₙ and D values from the SEC-measurements are overrated). The molecular weights (Mₙ or Mtop) and dispersities (D) of polymers F, G, N and O were determined with SEC (THF) against polystyrene standards.

Additionally, ¹H NMR in deuterated chloroform or DMSO-d₆ has been used to calculate the average number of 4H hydrogen bonding units per polymer chain by considering the integral of the benzylic signals of the polymer end group and the integral of the alkylidene signal of the 4H hydrogen bonding unit in case of polymers A-G and M. Polymers H-L and N contain incorporated 4H hydrogen bonding units, as evidenced by ¹H NMR data.

### Polymer properties

The effect of incorporation of 4H hydrogen bonding units in the polymer chain is clearly demonstrated in the solution (kinematic) viscosity of PMMA-samples at 20°C, see Table 2. As can be seen, incorporation of 4H hydrogen bonding units according to this invention has a pronounced and unique thickening effect.

**Table 2**

| Concentration in chloroform (g/L) | Kinematic viscosity polymer F (mm²/s) | Concentration in chloroform (g/L) | Kinematic viscosity polymer G (mm²/s) |
|---|---|---|---|
| 44 | 0.61 | 22 | 0.53 |
| 81 | 0.74 | 30 | 0.58 |
| 115 | 0.97 | 44 | 0.76 |
| 163 | 1.37 | 68 | 1.10 |
| 226 | 2.24 | 92 | 1.85 |
| 280 | 3.41 | 128 | 5.49 |

| | | | |
|---|---|---|---|
| polymer F and G have a comparable molecular weight, see table 1. | | | |

Incorporation of 4H hydrogen bonding units in polymers according to this invention also gives enhancement in material properties, as most clearly reflected in the difference in properties of polymers N and O. While poly(butylacrylate) O is a sticky viscous liquid, poly(butylacrylate) N with incorporated 4H hydrogen bonding units is an elastic material even despite the fact that polymer N has a lower molecular weight than polymer O.

## Claims

1. A monomer comprising (a) a monomeric unit having a group that can be polymerized (i.e. a monomeric unit having a polymerizable group) and having at least a functional group, a linking moiety (b) and a structural element (c) capable of forming at least four hydrogen bridges, wherein the monomer has the general structure (a) - (b) - (c), wherein the structural element (c) has the general formula (3) or (4), or tautomers thereof: 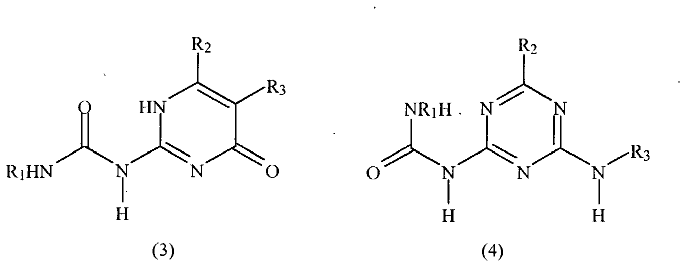 wherein the structural element (c) is bonded to the linking moiety (b) at R₁ with the other R groups representing a random side chain or are hydrogen atoms and wherein the functional group of the monomeric unit having a polymerizable group is selected from the group consisting of hydroxy, carboxylic acid, carboxylic ester, carboxylic acid halide, isocyanate, thioisocyanate, primary amine, secondary amine and halogen.

2. The monomer according to Claim 1, wherein the linking moiety (b) is a C₁-C₂₀ straight chain or branched alkylene, arylene, alkarylene or arylalkylene group.

3. The monomer according to Claim 1 or Claim 2, wherein R₂ is a random side chain and wherein R₃ is a hydrogen atom.

4. The monomer according to Claim 3, wherein the random side chain is methyl or ethylhexyl.

5. A process for the preparation of a monomer as defined in any one of Claims 1 - 4, wherein a monomeric unit having a polymerizable group is reacted in a first step with a compound having at least two functional groups and wherein the product obtained in the first step is reacted with a nitrogen containing compound, .

6. A process for the preparation of a monomer as defined in any one of Claims 1 - 4, wherein a nitrogen containing compound is reacted in a first step with a compound having at least two functional groups and wherein the product obtained in the first step is reacted with a monomeric unit having a polymerizable group.

7. A process for the preparation of a monomer as defined in any one of Claims 1 - 4, wherein a nitrogen containing compound is reacted directly with a monomeric unit having a polymerizable group and wherein the monomeric unit is able to form a urea linkage between both reactants.

8. A monomer as defined in any one of Claims 1 - 4 obtainable by the process according to any one of Claim 5 - 7.

9. A copolymer comprising the monomer as defined in any one of Claims 1 - 4 or 8 and at least one comonomer.

10. The copolymer according to Claim 9, wherein the comonomer is selected from the group of: acrylic acid; C₁-C₃₀ branched or linear alkyl esters of acrylic acid; methacrylic acid; C₁-C₃₀ branched or linear alkyl esters of methacrylic acid; acrylamides or methacrylamides wherein the amide group may be substituted with one or two C₁-C₃₀ branched or linear alkyl groups; vinyl esters, preferably vinyl acetates; other compounds having a vinyl group wherein said compounds are preferably selected from pyrrolidones, imidazoles, pyridines, caprolactams, piperidones, benzene and derivatives thereof; C₄-C₂₀ alkadienes; lactones; lactams; and saturated or unsaturated heterocyclic compounds containing one to five oxygen atoms.

11. The copolymer according to Claim 9 or Claim 10, wherein the copolymer is a linear, branched, star, hyperbranched, dendritic or comb-like copolymer

12. The copolymer according to any one of Claims 9 - 11, wherein the copolymer has a random, regular, tapered or block structure.

13. A process for the preparation of a copolymer, wherein a monomer as defined in any one of Claims 1 - 4 or 8 is polymerized with at least one comonomer.

14. The process according to claim 13, wherein the process is conducted in bulk, dispersion, solution, emulsion, suspension or inverse phase emulsion.

15. A copolymer obtainable by the process according to Claim 13 or Claim 14.

## Patentansprüche

1. Monomer, umfassend (a) eine Monomereinheit mit einer Gruppe, die polymerisiert werden kann (d. h. eine Monomereinheit mit einer polymerisierbaren Gruppe), und mit mindestens einer funktionellen Gruppe, eine Verknüpfungskomponente (b) und ein Strukturelement (c), das mindestens vier Wasserstoffbrücken ausbilden kann, wobei das Monomer die allgemeine Struktur (a) - (b) - (c) aufweist, wobei das Strukturelement (c) die allgemeine Formel (3) oder (4) aufweist, oder Tautomere davon: 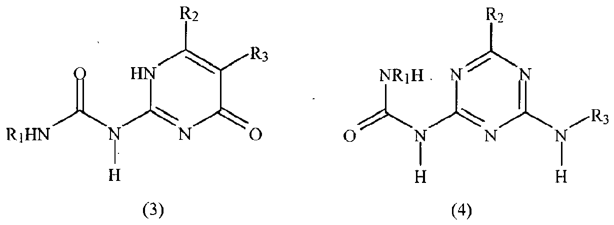 wobei das Strukturelement (c) über R₁ an die Verknüpfungskomponente (b) gebunden ist, wobei die anderen R-Gruppen eine beliebige Seitenkette verkörpern oder Wasserstoffatome sind und wobei die funktionelle Gruppe der Monomereinheit mit einer polymerisierbaren Gruppe ausgewählt ist aus der Gruppe bestehend aus Hydroxy, Carbonsäure, Carbonsäureester, Carbonsäurehalogenid, Isocyanat, Thioisocyanat, primärem Amin, sekundärem Amin und Halogen.

2. Monomer nach Anspruch 1, wobei die Verknüpfungskomponente (b) eine geradkettige oder verzweigte C₁-C₂₀-Alkylen-, C₁-C₂₀-Arylen-, C₁-C₂₀-Alkarylen- oder C₁-C₂₀-Aryl-alkylengruppe ist.

3. Monomer nach Anspruch 1 oder Anspruch 2, wobei R₂ eine beliebige Seitenkette ist und wobei R₃ ein Wasserstoffatom ist.

4. Monomer nach Anspruch 3, wobei die beliebige Seitenkette Methyl oder Ethylhexyl ist.

5. Verfahren zur Herstellung eines Monomers nach einem der Ansprüche 1 - 4, wobei eine Monomereinheit mit einer polymerisierbaren Gruppe in einem ersten Schritt mit einer Verbindung mit mindestens zwei funktionellen Gruppen umgesetzt wird und wobei das in dem ersten Schritt erhaltene Produkt mit einer stickstofflialtigen Verbindung umgesetzt wird.

6. Verfahren zur Herstellung eines Monomers nach einem der Ansprüche 1 - 4, wobei eine stickstoffhaltige Verbindung in einem ersten Schritt mit einer Verbindung mit mindestens zwei funktionellen Gruppen umgesetzt wird und wobei das in dem ersten Schritt erhaltene Produkt mit einer Monomereinheit mit einer polymerisierbaren Gruppe umgesetzt wird.

7. Verfahren zur Herstellung eines Monomers nach einem der Ansprüche 1 - 4, wobei eine stickstoffhaltige Verbindung direkt mit einer Monomereinheit mit einer polymerisierbaren Gruppe umgesetzt wird und wobei die Monomereinheit eine Harnstoffverknüpfung zwischen beiden Reaktanten ausbilden kann.

8. Monomer nach einem der Ansprüche 1 - 4, erhältlich mit dem Verfahren nach einem der Ansprüche 5 - 7.

9. Copolymer, umfassend das Monomer nach einem der Ansprüche 1 - 4 oder 8 und mindestens ein Comonomer.

10. Copolymer nach Anspruch 9, wobei das Comonomer ausgewählt ist aus der Gruppe aus: Acrylsäure; verzweigten oder linearen C₁-C₃₀-Alkylestem von Acrylsäure; Methacrylsäure; verzweigten oder linearen C₁-C₃₀-Alkylestem von Methacrylsäure; Acrylamiden oder Methacrylamiden, wobei die Amidgruppe mit ein oder zwei verzweigten oder linearen C₁-C₃₀-Alkylgruppen substituiert sein kann; Vinylestern, vorzugsweise Vinylacetaten; anderen Verbindungen mit einer Vinylgruppe, wobei die Verbindungen vorzugsweise ausgewählt sind aus Pyrrolidonen, Imidazolen, Pyridinen, Caprolactamen, Piperidonen, Benzol und Derivaten davon; C₄-C₂₀-Alkadienen, Lactonen; Lactamen und gesättigten oder ungesättigten heterocyclischen Verbindungen mit einem bis fünf Sauerstoffatomen.

11. Copolymer nach Anspruch 9 oder Anspruch 10, wobei das Copolymer ein lineares, verzweigtes, sternförmiges, hochverzweigtes, dendritisches oder kammförmiges Copolymer ist.

12. Copolymer nach einem der Ansprüche 9 - 11, wobei das Copolymer eine ungeordnete, regelmäßige, kegelförmige oder blockförmige Struktur aufweist.

13. Verfahren zur Herstellung eines Copolymers, wobei ein Monomer nach einem der Ansprüche 1 - 4 oder 8 mit mindestens einem Comonomer polymerisiert wird.

14. Verfahren nach Anspruch 13, wobei das Verfahren in Masse, Dispersion, Lösung, Emulsion, Suspension oder Umkehrphasenemulsion durchgeführt wird.

15. Copolymer, erhältlich mit dem Verfahren nach Anspruch 13 oder Anspruch 14.

## Revendications

1. Monomère comprenant (a) une unité monomère présentant un groupe pouvant être polymérisé (c'est à dire une unité monomère présentant un groupe polymérisable) et présentant au moins un groupe fonctionnel, un groupe de liaison (b) et un élément structural (c) capable de former au moins quatre ponts hydrogène, dans lequel le monomère présente la structure générale (a) - (b) - (c), dans lequel l'élément structural (c) présente la formule générale (3) ou (4), ou des tautomères de celle-ci : 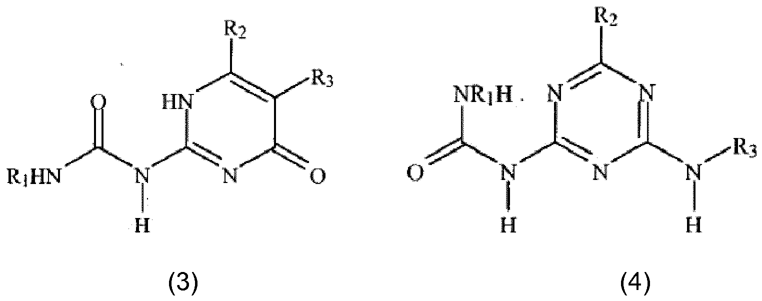 dans lequel l'élément de structure (c) est lié au groupe de liaison (b) au niveau de R₁ avec les autres groupes R représentant une chaîne latérale aléatoire ou sont des atomes d'hydrogène et dans lequel le groupe fonctionnel de l'unité monomère présentant un groupe polymérisable est choisi dans le groupe constitué par un groupe hydroxy, un acide carboxylique, un ester carboxylique, un halogénure d'acide carboxylique, un groupe isocyanate, un groupe thioisocyanate, un groupe amine primaire, un groupe amine secondaire et un d'halogène.

2. Monomère selon la revendication 1, dans lequel le groupe de liaison (b) est un groupe alkarylène, un groupe arylalkylène, un groupe arylène, un groupe alkylène, à chaîne linéaire ou ramifiée en C₁ à C₂₀.

3. Monomère selon la revendication 1 ou la revendication 2, dans lequel R₂ est une chaîne latérale aléatoire et dans lequel R₃ est un atome d'hydrogène.

4. Monomère selon la revendication 3, dans lequel la chaîne latérale aléatoire est un groupe méthyle ou un groupe éthylhexyle.

5. Procédé pour la préparation d'un monomère tel que défini dans l'une quelconque des revendications 1 à 4, dans lequel une unité monomère présentant un groupe polymérisable est mise en réaction dans une première étape avec un composé présentant au moins deux groupes fonctionnels et dans lequel le produit obtenu dans la première étape est mis en réaction avec un composé contenant de l'azote.

6. Procédé pour la préparation d'un monomère tel que défini dans l'une quelconque des revendications 1 à 4, dans lequel un composé contenant de l'azote est mis en réaction dans une première étape avec un composé présentant au moins deux groupes fonctionnels et dans lequel le produit obtenu dans la première étape est mis en réaction avec une unité monomère présentant un groupe polymérisable.

7. Procédé pour la préparation d'un monomère tel que défini dans l'une quelconque des revendications 1 à 4, dans lequel un composé contenant de l'azote est mis en réaction directement avec une unité monomère présentant un groupe polymérisable et dans lequel l'unité monomère est capable de former une liaison urée entre les deux réactifs.

8. Monomère tel que défini dans l'une quelconque des revendications 1 à 4 susceptible d'être obtenu par le procédé selon l'une quelconque des revendications 5 à 7.

9. Copolymère comprenant le monomère tel que défini dans l'une quelconque des revendications 1 à 4 ou 8 et au moins un comonomère.

10. Copolymère selon la revendication 9, dans lequel le comonomère est choisi dans le groupe constitué par : l'acide acrylique ; les esters alkyliques linéaires ou ramifiés en C₁ à C₃₀ de l'acide acrylique ; l'acide méthacrylique ; les esters alkyliques linéaires ou ramifiés en C₁ à C₃₀ de l'acide méthacrylique ; les acrylamides ou les méthacrylamides dans lesquels le groupe amide peut être substitué par un ou deux groupe(s) alkyle(s) linéaire(s) ou ramifié(s) en C₁ à C₃₀, les esters de vinyle, de préférence des acétates de vinyle; d'autres composés ayant un groupe vinyle dans lequel lesdits composés sont de préférence choisis parmi les pyrrolidones, les imidazoles, les pyridines, les caprolactames, les pipéridones, le benzène et des dérivés de ceux-ci ; les alcadiènes en C₄ à C₂₀ ; les lactones ; les lactames ; et des composés hétérocycliques saturés ou insaturés, contenant un à cinq atome(s) d'oxygène.

11. Copolymère selon la revendication 9 ou la revendication 10, dans lequel le copolymère est un copolymère linéaire, ramifié, étoilé, hyper-ramifié, dendritique ou en forme de peigne.

12. Copolymère selon l'une quelconque des revendications 9 à 11, dans lequel le copolymère présente une structure aléatoire, régulière, conique ou en bloc.

13. Procédé pour la préparation d'un copolymère, dans lequel un monomère tel que défini dans l'une quelconque des revendications 1 à 4 ou 8 est polymérisé avec au moins un comonomère.

14. Procédé selon la revendication 13, dans lequel le procédé est mené en masse, en dispersion, en solution, en émulsion, en suspension ou en émulsion en phase inverse.

15. Copolymère susceptible d'être obtenu par le procédé selon la revendication 13 ou la revendication 14.
